(19) Europäisches Patentamt
European Patent Office
Office européen des brevets

(11) Numéro de publication: **0 430 808 B1**

(12) # FASCICULE DE BREVET EUROPEEN

(45) Date de publication de fascicule du brevet: **29.03.95**

(51) Int. Cl.$^6$: **C07D 309/30**

(21) Numéro de dépôt: **90403394.1**

(22) Date de dépôt: **29.11.90**

(54) **Dérivés dihydropyranniques, leurs procédés de préparation et leur utilisation.**

(30) Priorité: **01.12.89 FR 8915869**

(43) Date de publication de la demande:
**05.06.91 Bulletin 91/23**

(45) Mention de la délivrance du brevet:
**29.03.95 Bulletin 95/13**

(84) Etats contractants désignés:
**AT BE CH DE DK ES FR GB GR IT LI LU NL SE**

(56) Documents cités:
**FR-A- 2 317 288**

(73) Titulaire: **RHONE-POULENC NUTRITION ANIMALE**
**Rue Marcel Lingot**
**F-03600 Commentry (FR)**

(72) Inventeur: **Chabardes, Pierre**
**24 rue Jeanne d'Arc**
**F-69110 Sainte-Foy Les Lyon (FR)**
Inventeur: **Duhamel, Lucette**
**32 rue Jacques Boutrolles**
**F-76130 Mont Saint Aignan (FR)**
Inventeur: **Duhamel, Pierre**
**32 rue Jacques Boutrolles**
**F-76130 Mont Saint Aignan (FR)**
Inventeur: **Guillemont, Jérôme**
**7 rue Rioult**
**F-27210 Beuzeville (FR)**
Inventeur: **Poirier, Jean-Marie**
**17 Impasse de la Grande Madeleine**
**F-76160 Saint Martin du Vivier (FR)**

(74) Mandataire: **Le Pennec, Magali et al**
**RHONE-POULENC RORER SA,**
**Direction des Brevets,**
**20 Avenue Raymond Aron**
**F-92165 Antony Cédex (FR)**

EP 0 430 808 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services
(3.10/3.09/3.3.3)

## Description

La présente invention concerne de nouveaux dérivés dihydropyranniques, leurs procédés de préparation et leur utilisation dans la préparation de diverses vitamines et notamment des vitamines A et E.

Il est connu selon les brevets J53-101308 et J53-101309 de préparer des intermédiaires de la vitamine A par hydrolyse de dérivés dihydropyranniques portant un radical alkyle en position 4 et éventuellement un groupe hydroxyle en position 2. Ces dérivés ne sont jamais substitués en position 6, leur procédé de préparation est réalisé par hydrogénation d'hydropéroxyde sur un catalyseur à base de palladium ou de platine. Ce procédé ne permet pas d'obtenir de dérivés pyranniques substitués en position 6 par une chaîne polyénique.

Il est encore connu selon le brevet FR 2317288 un procédé de préparation du rétinal ou d'intermédiaires de préparation de la vitamine A qui consiste à hydrolyser un dérivé alkoxydihydropyrannique en présence d'un catalyseur choisi parmi les acides sulfoniques ou carboxylique. Le dérivé alkoxydihydropyrannique est obtenu par condensation d'un alkoxydihydropyranne et d'un phosphonium par une réaction de Witting.

La présente invention par un procédé de préparation différent permet d'accéder à des dérivés pyranniques substitués en position 6 par divers radicaux dont les radicaux polyéthyléniques. Ces dérivés dihydropyranniques sont nouveaux, ils répondent à la formule (I) suivante :

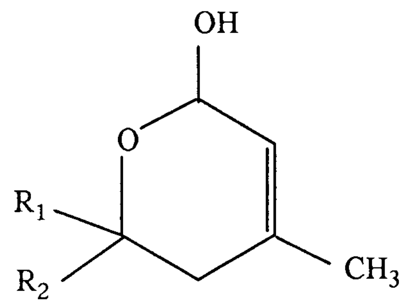

(I)

dans laquelle $R_1$ et $R_2$ identiques ou différents représentent l'hydrogène, un ou plusieurs groupes alkyle linéaire ou ramifié, alkényle linéaire ou ramifié, aryle ; les deux radicaux $R_1$ et $R_2$ ne pouvant simultanément représenter l'hydrogène.

Les radicaux alkyles, alkényles et aryles ont de préférence 1 à 18 atomes de carbone. On préfère tout particulièrement les dérivés de formule (I) pour lesquels $R_2$ représente l'hydrogène et $R_1$ un radical [méthyl-2 (triméthyl-2,6,6 cyclohexène-1) yl-4 butadiène] yl-6 de formule :

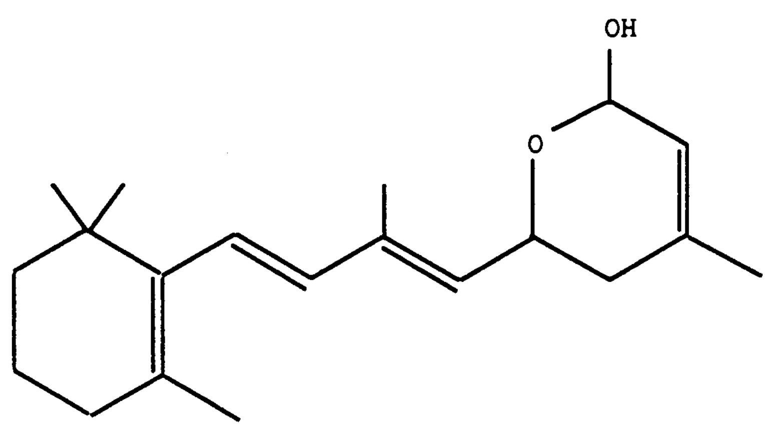

Ces composés de formule (I) sont préparés selon un procédé de mise en oeuvre qui consiste à mettre en présence le diénolate lithié du prénal de formule:

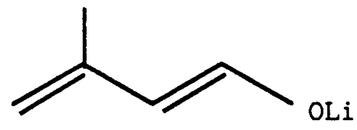

avec un aldéhyde de formule $R_1CHO$ ou une cétone de formule $R_1$-CO-$R_2$, les radicaux $R_1$ et $R_2$ ayant la même signification que précédemment. On préfère ainsi utiliser comme matière première la $\beta$-ionylidène acétaldéhyde et le diénolate lithié du prénal.

La condensation a lieu de préférence en présence d'un solvant inerte dans les conditions de réaction choisi parmi les éthers tels que tétrahydrofuranne, les hydrocarbures aliphatiques ou aromatiques tels que le pentane, l'hexane, le toluène, les xylènes.

La condensation a lieu de préférence à une tempéraure comprise entre -80 °C et +20 °C.

La préparation du diénolate lithié du prénal peut être réalisée par au moins deux méthodes qui consiste selon une première méthode à mettre en présence un (méthyl-3 butadiènoxy) silane de formule (II) suivante:

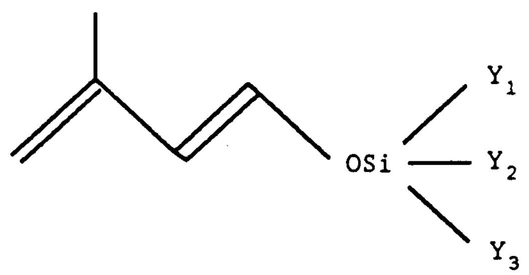

(II)

dans laquelle $Y_1$, $Y_2$, $Y_3$ identiques ou différents réprésentent un groupe alkyle contenant 1 à 4 atomes de carbone, de préférence un groupe méthyle, ou un groupe aryle contenant 6 à 12 atomes de carbone, de préférence un groupe phényle, avec un dérivé lithié de formule R'Li dans laquelle R' représente un groupe alkyle linéaire ou ramifié contenant 1 à 4 atomes de carbone, de préférence un groupe méthyle ou tertiobutyle.

La mise en contact est réalisée de préférence à une température comprise entre -80 °C et 0 °C.

La mise en contact est réalisée en l'absence de solvant ou dans un solvant inerte dans les conditions de réaction choisi parmi les éthers tels que le tétrahydrofuranne, les hydrocarbures aliphatiques ou aromatiques tels que le pentane, l'hexane, le toluène, les xylènes.

Le rapport molaire du dérivé lithié R'Li au composé de formule (II) est de préférence supérieur à 1 et tout particulièrement compris entre 1 et 1,2.

Selon une deuxième méthode de préparation du dérivé lithié, on met en présence un ester de méthyl-3 butadiénol répondant à la formule (III) :

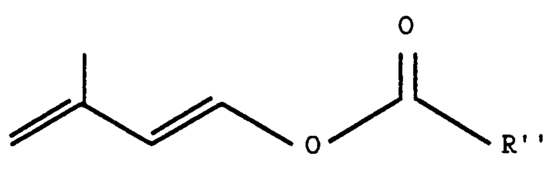

(III)

dans laquelle R'' représente un groupe alkyle linéaire ou ramifié contenant 1 à 6 atomes de carbone ou un groupe aryle contenant 6 à 12 atomes de carbone avec un dérivé lithié de formule R'Li dans laquelle R' a la même signification que précédemment.

Parmi les dérivés de formule (III) on préfère utiliser les dérivés pour lesquels R'' représente un groupe méthyle c'est-à-dire l'acétoxy-1 méthyl-3 butadiène.

La mise en contact du dérivé de formule (III) et du dérivé lithié est réalisée de préférence à une température comprise entre -80 °C et 0 °C.

La mise en contact est réalisée en l'absence de solvant ou dans un solvant inerte dans les conditions de réaction choisi parmi les éthers, de préférence le tétrahydrofuranne, les hydrocarbures aliphatiques ou

aromatiques tels que le pentane, l'hexane, le toluène, les xylènes.

Le rapport molaire du dérivé lithié R'Li au composé de formule (III) est de préférence supérieur à 2 et tout particulièrement compris entre 2 et 2,2.

Les composés de formule (I) sont utilisés pour la préparation d'intermédiaires terpéniques et de préférence pour la préparation du rétinal.

Les composés de formule (I) sont facilement hydrolysés en aldéhydes terpéniques par l'action d'un acide dans un solvant inerte dans les conditions de la réaction choisi parmi les éthers tels que le tétrahydrofuranne, les solvants aprotiques polaires tels que le diméthylformamide, la N-méthylpyrrolidone, les hydrocarbures aliphatiques ou aromatiques tels que le pentane, l'hexane, le toluène, les xylènes. On préfère utiliser comme solvant un mélange de diméthylformamide et de toluène.

La réaction peut être schématisée de la manière suivante :

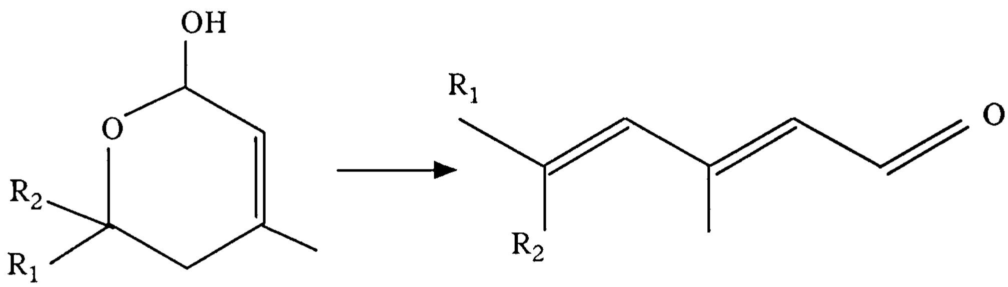

Il est avantageux d'ajouter un acide qui permet une catalyse de la réaction. Ces acides sont choisis de préférérence parmi:

- l'acide trichloroacétique,
- l'acide trifluoroacétique,
- l'acide trifluorométhanesulfonique,
- le chlorure de pyridinium,
- l'acide borique.

On préfère utiliser l'acide borique.

La quantité catalytique d'acide sera avantageusement comprise entre 0,01 et 0,05 mole par mole de dérivé de formule (I) à hydrolyser et de préférence égale à 0,02 mole par mole de dérivé (I).

Il est certain que l'ensemble des réactions formation du dérivé lithié, mise en contact avec la cétone ou l'aldéhyde et hydrolyse du cycle pyrannique peuvent être effectuées sans isoler les produits intermédiaires ("one pot synthesis").

La présente invention sera plus complètement décrite à l'aide des exemples suivants

EXEMPLE 1

Préparation des éthers d'énols silylés :

A une solution de 0,125 mol d' iodure de sodium (18,7 g) dans 130 ml d'acétonitrile, on ajoute 0,125 mol de chlorotriméthylsilane (13,6 g) et 0,125 mol de triéthylamine (12,6 g). On refroidit à une température inférieure à 10 °C et on ajoute, goutte à goutte, 0,1 mol d'aldéhyde. L'agitation est poursuivie de 3 à 12 heures à température ambiante. On filtre la suspension et on extrait par 5 fois 40 ml de pentane. La phase pentane est évaporée, puis le résidu distillé.

Méthyl-3 triméthylsiloxy-1 butadiène

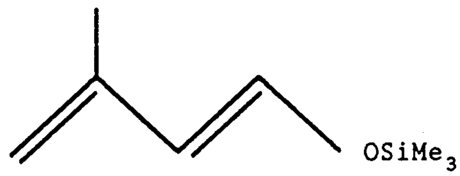

Eb : 48-50 ° C/18 mm Hg.
Rdt : 75 %.

EXEMPLE 2

Préparation de l'acétoxy-1 méthyl-3 butadiène-1,3

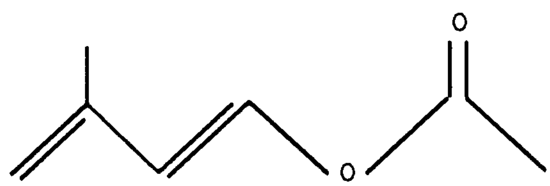

On coule progressivement 0,332 mole de prénal sur 0,532 mole d'acétate d'isopropényle dans lequel on a dissous 1 % en poids d'acide paratoluène sulfonique en distillant l'acétone au fur et à mesure de sa formation. La température de la masse est de 93-94 ° C et début et de 118 ° C après 1 heure 50 minutes de chauffage, l'acétoxy-1 méthyl-3 butadiène est ensuite purifié par distillation. Point d'ébullition 48-52 ° C/13 mm Hg.

EXEMPLE 3

CONDENSATION AVEC DES DERIVES CARBONYLES (Tableau 1)

1) Utilisation du méthyl-3 triméthylsiloxy-1 butadiène

Avec le benzaldéhyde : Phényl-6 méthyl-4 hydroxy-2 dihydro-2,5 2H pyranne

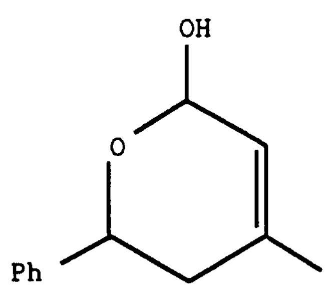

A une solution de 10 mmol d'éther de diénol silylé (1,56 g) dans 15 ml de tétrahydrofuranne (THF) anhydre, on ajoute à -20 ° C, 10 mmol de méthyllithium 2,5 N (4 ml) en 5 minutes. La réaction est exothermique et la solution se colore en jaune-orangé.

Après 15 minutes à -20 ° C, le milieu réactionnel est refroidi à -70 ° C pour introduire 10 mmol de benzaldéhyde (essai 3, tableau I) dans deux ml de THF. On laisse 15 minutes à -70 ° C. La température du milieu réactionnel est remontée progressivement à -20 ° C en 90 minutes. La solution est ensuite traitée à -20 ° C par 15 ml d'une solution aqueuse saturée d'hydrogénocarbonate de sodium. On extrait par 4 fois 15

ml d'éther, sèche sur sulfate de magnésium, filtre et évapore les solvants. Le produit brut de la réaction est purifié par chromatographie éclair.
Eluant : éther/éther de pétrole : 20/100.
Rdt:83 %. F = 90 °C. IR:3600-3200 ($\nu$ OH) ; 1600 ($\nu$ C=C).
Avec l'acétaldéhyde : Diméthyl-4,6 hydroxy-2 dihydro-2,5 2H pyranne

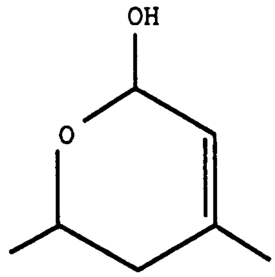

Eluant : éther/éther de pétrole : 20/100.
Rdt : 74 %. IR : 3600-3200 ($\nu$ OH) ; 1640 ($\nu$ C=C).
Avec le déhydrocitral : (Diméthyl-2,6 heptatriène)yl-6 méthyl-4 hydroxy-2 dihydro-2,5 2H pyranne

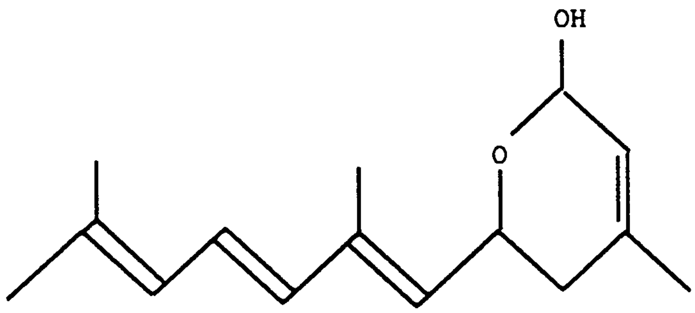

Eluant : éther/éther de pétrole : 20/100.
Rdt : 64,5 %. IR : 3600-3200 ($\nu$ OH); 1670 ($\nu$ C=C).
Avec l'hexadiénal : (Pentadiène-1,3)yl-6 méthyl-4 hydroxy-2 dihydro-2,5 2H pyranne

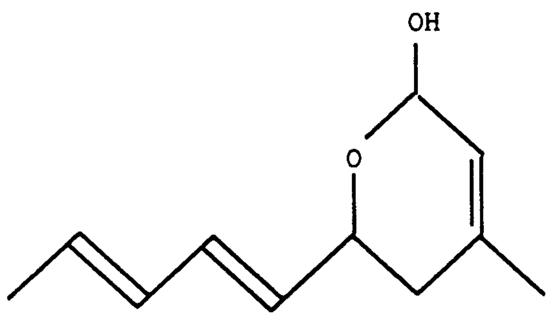

Eluant : éther/éther de pétrole : 20/100.
Rdt : 45 %. IR : 3600-3200 ($\nu$ OH); 1670 ($\nu$ C=C).
Avec le $\beta$-ionylidènacétaldéhyde : méthyl-2 (triméthyl-2,6,6 cyclohexène-1)yl-4 butadiène)yl-6 méthyl-4 hydroxy-2 dihydro-2,5 2H pyranne.

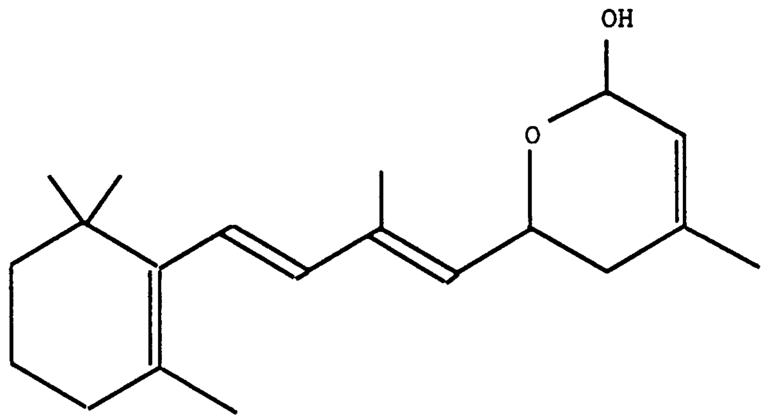

A une solution de 2,4 mmol de méthyl-3 triméthylsiloxy-1 butadiène (0,38 g) dilué dans 5 ml de tétrahydrofuranne anhydre, on ajoute sous argon, goutte à goutte à -20 °C, 2,4 mmol de n-butyllithium 2,5 N (0,96 ml) ou 2,4 mmol de méthyllithium 2,5 N (0,96 ml). Après une demi-heure de contact à -20 °C, la solution devenue jaune est refroidie à -45 °C. 2 mmol de $\beta$-ionylidène acétaldéhyde (O,436 g) en solution dans 2 ml de THF anhydre sont introduites en 5 minutes à -45 °C. On laisse sous agitation 3 heures à cette température. Le degré d'avancement de la réaction est contrôlé en CCM.

On traite le milieu réactionnel à -45 °C par 7 ml d'une solution aqueuse saturée d'hydrogénocarbonate de sodium. On extrait par 4 fois 10 ml d'éther et sèche la phase éthérée sur sulfate de magnésium pendant une heure. Après filtration on évapore les solvants.

Le produit brut est purifié par chromatographie éclair. Nous utilisons une colonne de 5 mm de diamètre et environ 20 g de silice pour 0,5 g de produit brut. On isole successivement le $\beta$-ionylidène acétaldéhyde (25 %) n'ayant pas réagi, puis le dérivé pyrannique en "C20".

Eluant : éther/éther de pétrole : 20/100.

Rdt : 66 %. IR : 3600-3200 : ($\nu$ OH) ; 1650 ($\nu$ C=C).

2) Utilisation de l'acétoxy-1 méthyl-3 butadiène

Avec l'isovaléraldéhyde : Isobutyl-6 méthyl-4 hydroxy-2 dihydro-2,5 2H pyranne

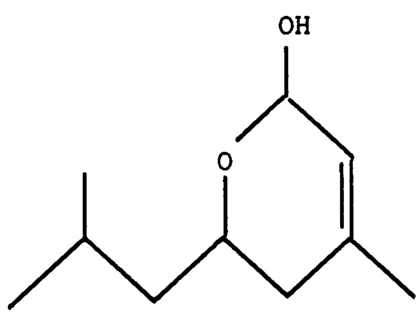

A une solution de 10 mmol de méthyl-3 acétoxy-1 butadiène dans 15 ml de THF anhydre, on ajoute à -30 °C en 5 minutes, 20 mmol de méthyllithium 1,6 N (12,5 ml). Après 15 minutes à -30 °C, le milieu réactionnel est refroidi à -70 °C pour introduire 8,5 mmol d'isovaléraldéhyde dans 2 ml de THF. On remonte la température à 20 °C en une heure. On traite à -40 °C le milieu réactionnel par 15 ml d'une solution aqueuse saturée d'hydrogénocarbonate de sodium. On extrait par 4 fois 15 ml d'éther, sèche sur sulfate de magnésium, filtre et évapore les solvants. le produit brut de la réaction est purifié par chromatographie éclair.

Eluant : éther/éther de pétrole : 20/100.

Rdt : 74 %. IR : 3600-3200 ($\nu$ OH) ; 1650 ($\nu$ C=C).

Avec l'acétone : Triméthyl-4,6,6 hydroxy-2 dihydro-2,5 2H pyranne

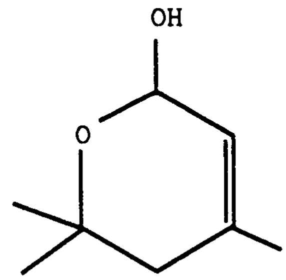

Eluant : éther/éther de pétrole : 20/100.
Rdt : 25 %. IR : 3600-3200 (ν OH) ; 1650 (ν C=C).

L'ensemble des résultats est indiqué dans le tableau 1.

EXEMPLE 4

Transformation des dérivés pyranniques en aldéhydes polyéniques (tableau 2 et 3)

Rétinal (Tableau 2)

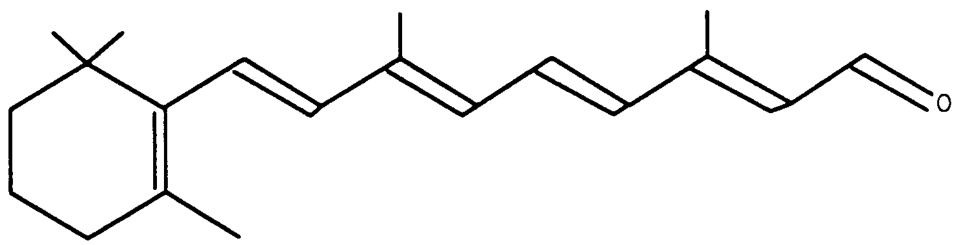

Sous atmosphère inerte, dans un bicol de 25 ml muni d'un thermométre et d'un réfrigérant, on introduit 1 mmol de dérivé pyrannique (0,3 g) dilué dans un mélange de 1,2 ml de diméthylformamide (DMF) et de 4,8 ml de toluène, puis 0,09 mmol d'acide borique (5,6 ml) en solution dans 1 ml de DMF (voir tableau 2, essai 4).

Le mélange réactionnel est placé dans un bain d'huile préalablement chauffé à 110°C. Après 15 minutes à cette température, la tâche du dérivé pyrannique en "C20" a totalement disparu en CCM. La solution est alors refroidie jusqu'à température ambiante, puis est neutralisée par 5 ml d'une solution aqueuse saturée d'hydrogénocarbonate de sodium. On extrait la phase aqueuse par 4 fois 10 ml d'éther, puis on sèche la phase éthérée sur sulfate de magnésium et on concentre les solvants en présence de ionol. Le produit est purifié par chromatographie éclair sur silice.
Eluant : éther/éther de pétrole : 5/100).
Le rétinal est isolé avec un rendement de 60 %.
IR : 1665 (ν C=O) ; 1580 (ν C=C)

Dans les mêmes conditions, en utilisant 0,09 mmol de chlorure de pyridinium (10 mg), on obtient le rétinal avec un rendement de 65 % (Tableau 2, essai 3).

Les différents résultats pour la préparation du rétinal sont indiqués dans le tableau 2.

Les différents aldéhydes polyéniques préparés par hydrolyse de dérivés pyranniques de formule (I), en utilisant comme catalyseur le chlorure de pyridinium, dans les conditions de l'essai 3, tableau 2, sont indiqués dans le tableau 3.

TABLEAU 1

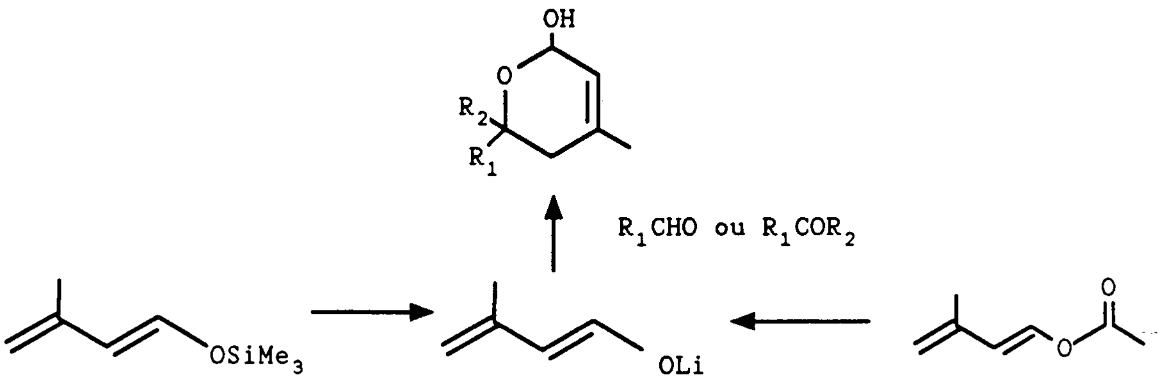


| ESSAI | $R_1CHO$ ou $R_1COR_2$ | PRODUIT DE LA REACTION | RENDEMENT Origine éther énol silylé | RENDEMENT Origine acétate d'énol |
|---|---|---|---|---|
| 1 | 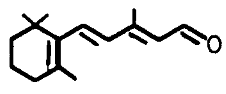 | 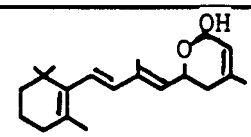 | 66 | 60 |
| 2 | 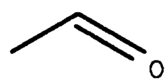 | 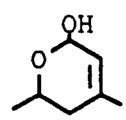 | 74 | |
| 3 | 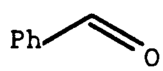 | 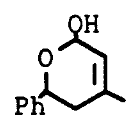 | 83 | 94 |
| 4 | 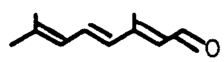 | 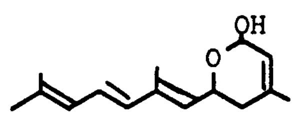 | 75,5 | |
| 5 | 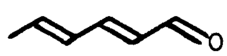 | 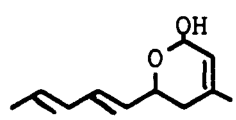 | 45 | 50 |
| 6 | 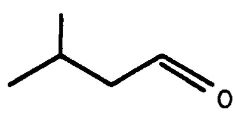 | 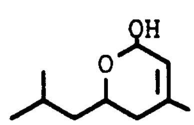 | | 74 |
| 7 | 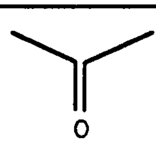 | 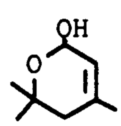 | | 25 |

TABLEAU 2 : Préparation du rétinal

| ESSAIS | CATALYSEURS % en mol | | T°C | DUREE (h) | RENDEMENTS % RETINAL |
|---|---|---|---|---|---|
| 1 | $CCl_3CO_2H$ | 2 | 80 | 0,25 | 25 |
| 2 | $CF_3CO_2H$ | 2 | T.A | 18 | 45 |
| 3 | 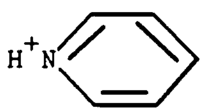 , $Cl^-$ | 1 | 110 | 0,25 | 65 |
| 4 | $H_3BO_3$ | 1 | 110 | 0,25 | 60 |

Le rendement est exprimé en produit purifié par chromatographie éclair.

TABLEAU 3

PREPARATION DES

ALDEHYDES POLYETHYLENIQUES

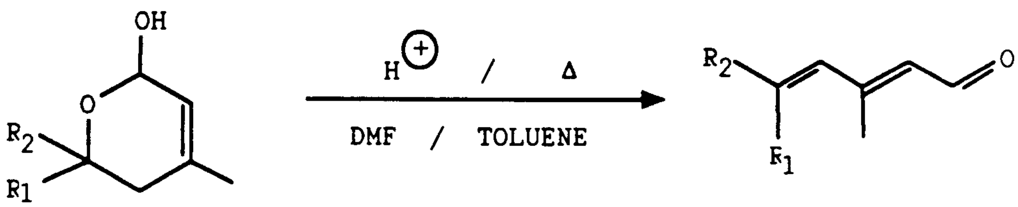


| Essais | Dérivés pyranniques | Aldéhydes | Rdt % |
|---|---|---|---|
| 1 | 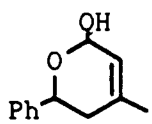 | 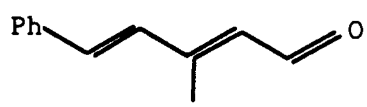 | 70 |
| 2 | 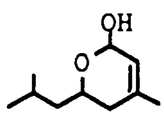 | 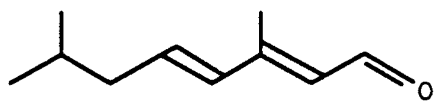 | 63 |
| 3 | 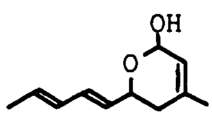 | 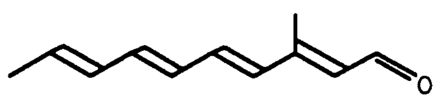 | 69 |
| 4 | 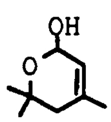 | 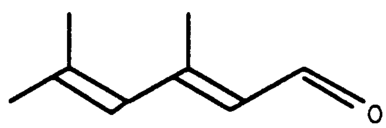 | 30 |
| 5 | 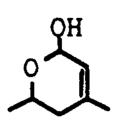 | 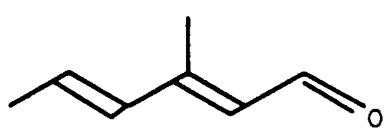 | 55 |
| 6 | 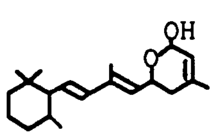 | 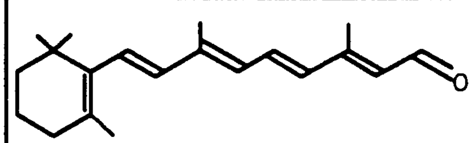 | 65 |

Catalyseur : chlorure de pyridinium

## Revendications

**1.** Procédé de préparation de dérivés dihydropyranniques répondant à la formule (I) :

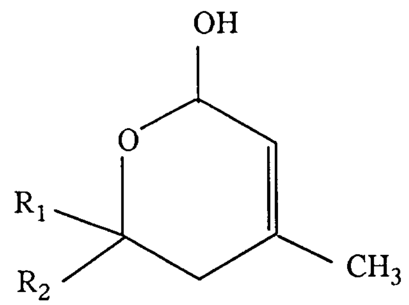

(I)

dans laquelle $R_1$ et $R_2$ représentent un ou plusieurs radicaux identiques ou différents choisis parmi les groupes hydrogène, alkyle linéaire ou ramifié, alkényle linéaire ou ramifié, aryle, contenant 1 à 18 atomes de carbone, caractérisé en ce que l'on met en présence le diénolate lithié du prénal avec un aldéhyde ou une cétone de formule $R_1CHO$ ou $R_1COR_2$ dans lesquelles $R_1$ et $R_2$ ont la même signification que précédemment et ne représentent pas simultanément l'hydrogène.

**2.** Procédé de préparation de dérivés selon la revendication 1 caractérisé en ce que $R_1CHO$ représente le $\beta$-ionylidène acétaldéhyde de formule

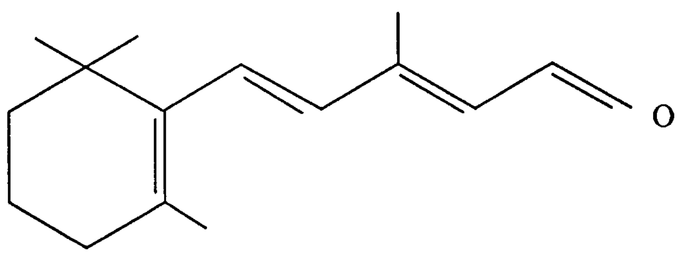

**3.** Procédé de préparation de dérivés dihydropyraniques selon la revendication 1 caractérisé en ce que le diénolate lithié du prénal est obtenu en mettant en présence le méthyl-3 butadiènoxy silane de formule (II) suivante :

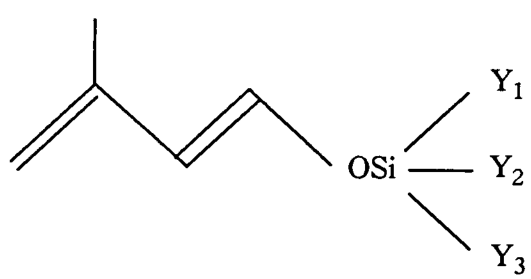

(II)

dans laquelle $Y_1$, $Y_2$, $Y_3$ identiques ou différents représentent un groupe alkyle, de préférence un groupe méthyle, ou un groupe aryle, de préférence un groupe phényle, avec un dérivé lithié de formule R'Li dans laquelle R' représente un groupe alkyle linéaire ou ramifié, de préférence un groupe méthyle ou tertiobutyle.

**4.** Procédé de préparation des dérivés dihydropyraniques selon la revendication 1 caractérisé en ce que le diénolate lithié du prénal est obtenu en mettant en présence un ester de méthyl-3 butadiénol

répondant à la formule (III) :

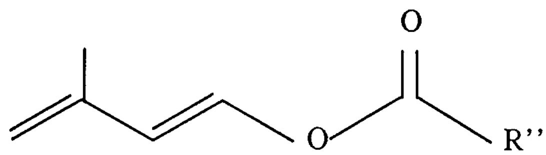

(III)

dans laquelle R'' représente un groupe alkyle linéaire ou ramifié contenant 1 à 6 atomes de carbone ou un groupe aryle avec un dérivé lithié de formule R'Li dans laquelle R' a la même signification que précédemment.

**5.** Procédé selon la revendication 3 caractérisé en ce qu'on met en présence une quantité de dérivé lithié R'Li telle que le rapport molaire R'Li/dérivé de formule (II) soit compris entre 1 et 1,2.

**6.** Procédé selon la revendication 4 caractérisé en ce qu'on met en présence une quantité de dérivé lithié R'Li telle que le rapport molaire R'Li/dérivé de formule (III) soit compris entre 2 et 2,2.

## Claims

**1.** Process for the preparation of dihydropyran derivatives corresponding to the formula (I):

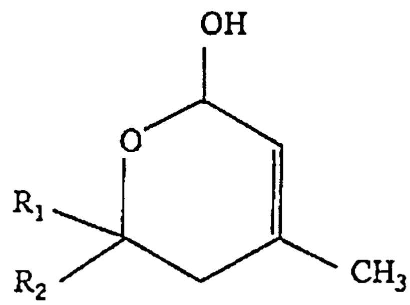

(I)

in which $R_1$ and $R_2$ represent one or more radicals, which may be identical or different, chosen from hydrogen or linear or branched alkyl groups, linear or branched alkenyl groups or aryl groups, containing 1 to 18 carbon atoms, characterized in that the lithiated dienolate of prenal is placed together with an aldehyde or a ketone of formula $R_1CHO$ or $R_1COR_2$ in which $R_1$ and $R_2$ have the same meaning as above and do not simultaneously represent hydrogen.

**2.** Process for the preparation of derivatives according to claim 1, characterized in that $R_1CHO$ represents $\beta$-ionylideneacetaldehyde, of formula

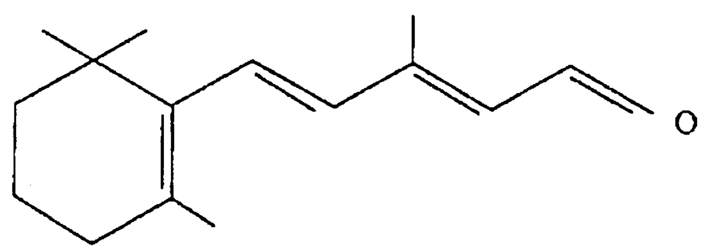

**3.** Process for the preparation of dihydropyran derivatives according to claim 1, characterized in that the lithiated dienolate of prenal is obtained by placing the 3-methylbutadieneoxysilane of following formula (II):

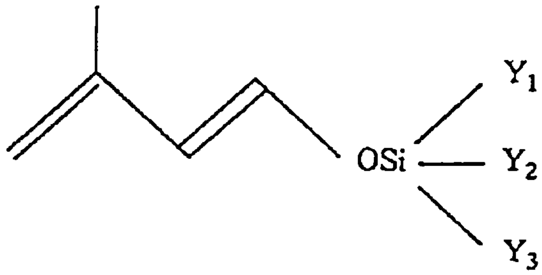

(II)

in which $Y_1$, $Y_2$ and $Y_3$, which may be identical or different, represent an alkyl group, preferably a methyl group, or an aryl group, preferably a phenyl group, together with a lithiated derivative of formula R'Li in which R' represents a linear or branched alkyl group, preferably a methyl or tert-butyl group.

4. Process for the preparation of the dihydropyran derivatives according to claim 1, characterized in that the lithiated dienolate of prenal is obtained by placing an ester of 3-methylbutadienol corresponding to the formula (III):

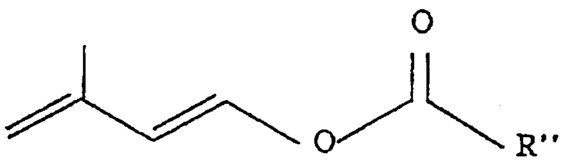

(III)

in which R" represents a linear or branched alkyl group containing 1 to 6 carbon atoms or an aryl group, together with a lithiated derivative of formula R'Li in which R' has the same meaning as above.

5. Process according to claim 3, characterized in that an amount of lithiated derivative R'Li is provided such that the R'Li/derivative of formula (II) molar ratio is between 1 and 1.2.

6. Process according to claim 4, characterized in that an amount of lithiated derivative R'Li is provided such that the R'Li/derivative of formula (III) molar ratio is between 2 and 2.2.

**Patentansprüche**

1. Verfahren zur Herstellung von Dihydropyranderivaten der Formel (I):

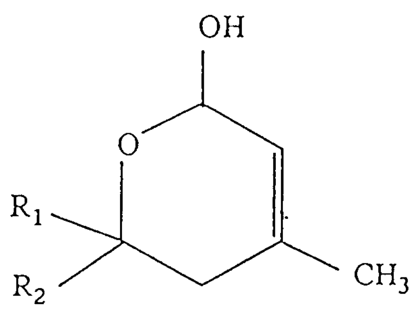

(I)

worin $R_1$ und $R_2$ einen oder mehrere identische oder verschiedene Reste bedeuten, ausgewählt unter Wasserstoff, linearem oder verzweigtem Alkyl, linearem oder verweigtem Alkenyl, Aryl, enthaltend 1 bis 18 Kohlenstoffatome, dadurch gekennzeichnet, daß man Prenal-lithium-dienolat mit einem Aldehyd oder einem Keton der Formel $R_1CHO$ oder $R_1COR_2$, worin $R_1$ und $R_2$ die vorstehend angegebene Bedeutung besitzen und nicht gleichzeitig für Wasserstoff stehen, in Kontakt bringt.

2. Verfahren zur Herstellung von Derivaten gemäß Anspruch 1, dadurch gekennzeichnet, daß $R_1CHO$ für den $\beta$-Ionyliden-acetaldehyd der Formel

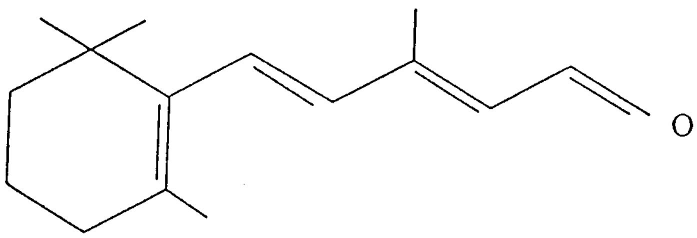

steht.

3. Verfahren zur Herstellung von Dihydropyranderivaten gemäß Anspruch 1, dadurch gekennzeichnet, daß das Prenal-lithium-dienolat erhalten wird, indem man 3-Methyl-butadienoxy-silan der folgenden Formel (II):

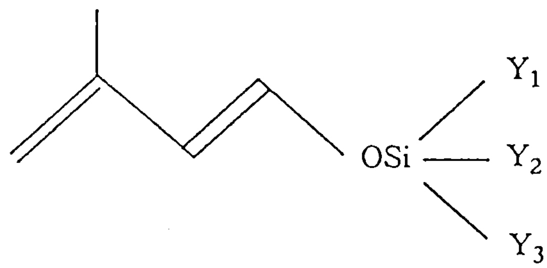

(II)

worin $Y_1$, $Y_2$, $Y_3$, die identisch oder voneinander verschieden sind, eine Alkylgruppe, vorzugsweise eine Methylgruppe, oder eine Arylgruppe, vorzugsweise eine Phenylgruppe, bedeuten, mit einem Lithiumderivat der Formel R'Li, worin R' für eine lineare oder verzweigte Alkylgruppe, vorzugsweise eine Methyl- oder tert.-Butylgruppe steht, in Kontakt bringt.

4. Verfahren zur Herstellung von Dihydropyranderivaten gemäß Anspruch 1, dadurch gekennzeichnet, daß das Prenal-lithium-dienolat erhalten wird, indem man einen Ester von 3-Methyl-butadienol der Formel (III):

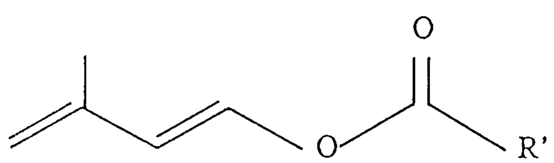

(III)

worin R'' eine lineare oder verzweigte Alkylgruppe mit 1 bis 6 Kohlenstoffatomen oder eine Arylgruppe bedeutet, mit einem Lithiumderivat der Formel R'Li, worin R' die vorstehend angegebene Bedeutung besitzt, in Kontakt bringt.

5. Verfahren gemäß Anspruch 3, dadurch gekennzeichnet, daß man eine derartige Menge des Lithiumderivats R'Li einsetzt, daß das Molverhältnis R'Li/Derivat der Formel (II) zwischen 1 und 1,2 liegt.

6. Verfahren gemäß Anspruch 4, dadurch gekennzeichnet, daß man eine derartige Menge des Lithiumderivats R'Li einsetzt, daß das Molverhältnis R'Li/Derivat der Formel (III) zwischen 2 und 2,2 liegt.